# EUROPEAN PATENT APPLICATION

(11) **EP 4 624 448 A1**
(43) Date of publication of application: **01.10.2025**
(21) Application number: 24791809.7
(22) Date of filing: 22.03.2024
(51) Int. Cl.: C07C 43/215, C07C 41/18, A61K 31/09, A61P 25/00, A61P 25/08, A61P 25/16

(54) **1-(CYCLOBUTYLIDENEMETHYL)-2,4,5-TRIMETHOXYBENZENE COMPOUND, PREPARATION METHOD THEREFOR AND USE THEREOF**

(30) Priority: 20.04.2023 CN 202310430869
(71) Applicant: CHENGDU XINRUI TAIKANG TECHNOLOGY CO., LTD., Chengdu, Sichuan 610200 (CN)
(72) Inventor: MAO, Shengjun, Chengdu, Sichuan 610041 (CN); LI, Rui, Chengdu, Sichuan 610041 (CN); ZHANG, Jian, Chengdu, Sichuan 610041 (CN); LIAO, Can, Chengdu, Sichuan 610041 (CN); YAN, Ruijie, Chengdu, Sichuan 610041 (CN); ZHANG, Di, Chengdu, Sichuan 610041 (CN); LI, Kai, Chengdu, Sichuan 610041 (CN); LIU, Qi, Chengdu, Sichuan 610041 (CN)
(74) Representative: Bayramoglu et al.
(86) International application number: PCT/CN2024/083221
(87) International publication number: WO 2024/217222

(57) **Abstract**

A 1-(cyclobutylidenemethyl)-2, 4, 5-trimethoxybenzene compound and a preparation method and use thereof are provided, belonging to the field of drug development technology. The prepared 1-(cyclobutylidenemethyl)-2, 4, 5-trimethoxybenzene compound may be used for the preparation of an antiepileptic drug and drugs for treatment and/or prevention of traumatic craniocerebral injury disorders, ischemic stroke, hemorrhagic stroke, and Parkinson's disease.

## Description

Claims 1 to 10 of the present invention were filed on April 20, 2023, with the application number CN 202310430869.9, and the invention is titled "1-(cyclobutanemethylidene)-2, 4, 5-trimethoxybenzene compound and preparation method and use thereof " for the priority of the patent application.

### TECHNICAL FIELD

The present disclosure relates to the technical field of drug research and development, and in particular, to 1-(cyclobutylidenemethyl)-2, 4, 5-trimethoxybenzene compound and preparation method and use thereof.

### BACKGROUND

Spreading depolarization (SD) is defined as a fluctuation in the gray matter of the central nervous system characterized by neuronal swelling, dendritic spine deformation, large changes in slow potential, and suppression of cerebral electrical activities (spreading cerebral electrical activities). At present, clinical electrophysiological evidence has demonstrated the prevalence of diffusion depolarization in individuals with migraine aura, ischemic stroke, traumatic brain injury, aneurysmal subarachnoid hemorrhage (aSAH) and delayed cerebral ischemia (DCI) following subarachnoid hemorrhage, spontaneous cerebral hemorrhage, subdural hematoma, spontaneous intracranial hemorrhage or traumatic brain injury, Alzheimer' s disease, Parkinson' s disease, epilepsy, etc. Therefore, diffusion depolarization represents a major, and yet frequently disregarded, pathophysiological process in the domain of acute neurology. Diffusion depolarization may be experimentally induced by a plurality of deleterious conditions such as potassium, glutamate, sodium-pumping inhibitors, epileptic status epilepticus, hypoxia, hypoglycemia, ischemia, etc., but it may also attack healthy, unaffected affected tissues. The physiologic hemodynamic response generated by the resistance vascular effect may lead to transient hyperperfusion in healthy tissues or a reverse hemodynamic response or diffuse ischemia in tissues undergoing progressive injury leading to severe hypoperfusion, thereby accelerating lesion progression. Therapies targeting diffusion depolarization or reverse hemodynamic response may therefore potentially be useful in treating these neurological disorders. Therefore, it is of great significance to provide a 1-(cyclobutylidenemethyl)-2, 4, 5-trimethoxybenzene compound, and a preparation method and use thereof, to effectively inhibit diffusion depolarization in a variety of neurological disorders as described above.

### SUMMARY

The object of the present disclosure is to provide a 1-(cyclobutylidenemethyl)-2, 4, 5-trimethoxybenzene compound and a preparation method and use thereof, in order to solve the problem that commercially available drugs in the prior art are not effective in inhibiting diffusion depolarization in neurological disorders.

In order to realize the above objects of the present disclosure, the present disclosure provides the following technical solution.

The present disclosure provides a 1-(cyclobutylidenemethyl)-2, 4, 5-trimethoxybenzene compound, the 1-(cyclobutylidenemethyl)-2, 4, 5-trimethoxybenzene compound having a structural formula (I):

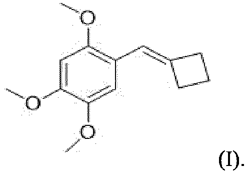

The present disclosure provides a method for preparing the 1-(cyclobutylidenemethyl)-2, 4, 5-trimethoxybenzene compound, including the following operations.
(1) mixing a compound 1a with dichloromethane to obtain a mixture, and adding a compound 1b to the mixture for reaction to obtain a compound 1c.
(2) mixing the compound 1c, compound 1d, and aluminum trichloride with methylene chloride in turn for reaction to obtain a compound 1e.
(3) mixing the compound 1e, sodium borohydride solution, and sodium hydroxide solution with tetrahydrofuran in turn for reaction to obtain a compound 1f.
(4) mixing the compound 1f, sodium acetate, and acetic anhydride for reaction to obtain the 1-(cyclobutylidenemethyl)-2, 4, 5-trimethoxybenzene compound.

The compound 1a is 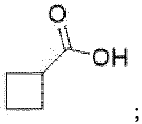 the compound 1b is 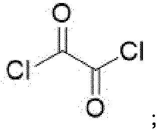 and the compound 1d is

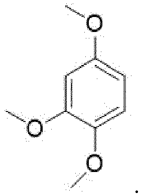

The present disclosure provides use of the 1-(cyclobutylidenemethyl)-2, 4, 5-trimethoxybenzene compound in preparation of an antiepileptic drug.

The present disclosure provides use of the 1-(cyclobutylidenemethyl)-2, 4, 5-trimethoxybenzene compound in preparation of a drug for treatment and/or prevention of traumatic craniocerebral injury disorders.

The present disclosure provides use of the 1-(cyclobutylidenemethyl)-2, 4, 5-trimethoxybenzene compound in preparation of a drug for treatment and/or prevention of ischemic stroke.

The present disclosure provides use of the 1-(cyclobutylidenemethyl)-2, 4, 5-trimethoxybenzene compound in preparation of a drug for treatment and/or prevention of hemorrhagic stroke.

The present disclosure provides use of the 1-(cyclobutylidenemethyl)-2, 4, 5-trimethoxybenzene compound in preparation of a drug for prevention and/or treatment of Parkinson's disease.

The 1-(cyclobutylidenemethyl)-2, 4, 5-trimethoxybenzene compound (hereinafter referred to as CTMB) provided by the present disclosure may be made into different dosage forms by adding pharmaceutically acceptable excipients, for example, which may be made into liquid dosage forms such as emulsion injections, oral emulsions, etc. In addition, technicians in the field may also prepare the CTMB into solid dosage forms such as tablets, capsules, etc., by conventional technical means. The dosage forms mentioned above are administered via intravenous injection, intramuscular injection, oral administration, etc., as long as it ensures that the compound of the structural formula (I) of the present disclosure reaches the site of action and achieves an effective concentration.

Beneficial effects of the present disclosure are as follows.
(1) CTMB of the present disclosure is capable of reducing the grade and incidence of seizures caused by pentylenetetrazole and significantly prolonging the latency period of epileptic clonic seizures and tonic seizures, and the effect of CTMB is a dose-dependent. Further, CTMB is also capable of significantly reducing the grade of seizures in the acute phase of a rat epilepsy model caused by lithium-pilocarpine and improving the survival rate of the rats, and the effect is superior to that of sodium valproate of a broad-spectrum antiepileptic drug.
(2) The drug prepared by CTMB significantly improves the neurobehavioral function of the rats with ischemic stroke and hemorrhagic stroke and restores the sense and motor function of the rats.
(3) CTMB improves the integrity of the blood-brain barrier, promotes the recovery of motor function in model rats; reduces neuroinflammatory responses, antagonizes glutamate excitatory neurotoxicity, reduces neuronal apoptosis, and improves neurological deficits and emotional anxiety in the model rats, which has a significant neuroprotective effect on traumatic craniocerebral injury.
(4) For model mice with Parkinson's disease, CTMB improves the motor function, improves its coordination and balance ability, increases the number of nigrosomes in the substantia nigra region of the model mice, reduces neuronal damage in the striatum and substantia nigra region, reduces the concentration of malondialdehyde (MDA) in the striatum of the model mice, and increases the concentration of glutathione (GSH) and the activity of superoxide dismutase (SOD) in the striatum of the model mice, which in turn improves the oxidative stress response of the striatum.

### BRIEF DESCRIPTION OF THE DRAWINGS

FIG. 1 is a diagram illustrating a survival situation of squeeze-and-excitation (SE) model rats after drug intervention in the acute phase;
FIG. 2 is a diagram illustrating a result of a blood-brain barrier permeability assay according to test 3 (a content of Evans blue dye per gram of brain tissue in different groups of rats);
FIG. 3 is a diagram illustrating a result of TNF- α , IL-1, and IL-6 assays in cerebral cortex of rats according to test 4;
FIG. 4 is a diagram illustrating a result of GLT-1, GABA, and GLT-1/GABA assays for different dosing groups according to test 5;
FIG. 5 is a hematoxylin-eosin (HE) staining picture of a substantia nigra (SN) of midbrain of mice;
FIG. 6 is a HE staining picture of striatum (STR) neurons of mice, where the position indicated by green arrow represents necrotic neurons and the position indicated by yellow arrow represents proliferating glial cells;
FIG. 7 is a staining picture of Nissl bodies of neurons in substantia nigra region of midbrain of mice, where the position indicated by arrow represents Nissl bodies; and
FIG. 8 is a diagram illustrating an effect of administration of 1-(cyclobutylidenemethyl)-2, 4, 5-trimethoxybenzene (CTMB) on tyrosine hydroxylase (TH) protein expression in substantia nigra region of midbrain of mice.

### DETAILED DESCRIPTION OF THE EMBODIMENTS

The present disclosure provides a 1-(cyclobutylidenemethyl)-2, 4, 5-trimethoxybenzene compound, the 1-(cyclobutylidenemethyl)-2, 4, 5-trimethoxybenzene compound having a structural formula (I):

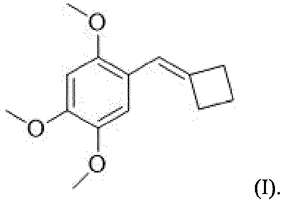

The present disclosure provides a method for preparing the 1-(cyclobutylidenemethyl)-2, 4, 5-trimethoxybenzene compound, which may include the following operations.
(1) A compound 1a is mixed with dichloromethane to obtain a mixture, and a compound 1b is added to the mixture for reaction to obtain a compound 1c.
(2) The compound 1c, compound 1d, and aluminum trichloride are mixed with methylene chloride in turn for reaction to obtain a compound 1e.
(3) The compound 1e, sodium borohydride solution, and sodium hydroxide solution are mixed with tetrahydrofuran in turn for reaction to obtain a compound 1f.
(4) The compound 1f, sodium acetate, and acetic anhydride are mixed for reaction to obtain the 1-(cyclobutylidenemethyl)-2, 4, 5-trimethoxybenzene compound.

The compound 1a is 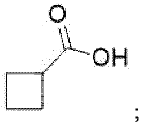 the compound 1b is 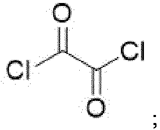 and the compound 1d is

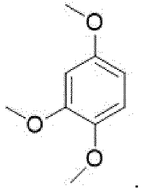

In the present disclosure, in operation (1), a mass-volume-mass ratio of the compound 1a, dichloromethane, and the compound 1b (also referred to a ratio of a mass of the compound 1a, a volume of dichloromethane, and a mass of the compound 1b) is within a range of (23-27) g: (130-170) mL: (93-97) g, preferably within a range of (24-26) g: (140-160) mL: (94-96) g, further preferably 25 g: 150 mL: (94.5-95.5) g.

In the present disclosure, the operation of mixing compound 1a with methylene chloride is preferably carried out under a nitrogen-protected condition, and the compound 1b is preferably added drop-by-drop when mixing with methylene chloride.

In the present disclosure, in operation (1), a reaction temperature of the reaction is within a range of 20-30°C, preferably within a range of 22-28°C, further preferably 25°C. The reaction is carried out under a stirring condition and the reaction is ended until no bubble is generated under the stirring condition.

In the present disclosure, in operation (2), a mass-mass-mass-volume ratio of the compound 1c, the compound 1d, aluminum trichloride, and methylene chloride (also referred to a ratio of a mass of the compound 1c, a mass of the compound 1d, a mass of aluminum trichloride, and a volume of methylene chloride) is within a range of (22-27) g: (30-33) g: (26-30) g: (80-120) mL, preferably within a range of (23-26) g: (30.5-32.5) g: (27-29) g: (85-105) mL, further preferably within a range of (23.5-25.5) g: (30.7-32.3) g: (27.5-28.5) g: (90-100) mL.

The compound 1c is

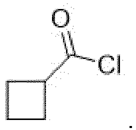

In the present disclosure, the aluminum trichloride is mixed at a temperature within a range of -2 to 2°C, preferably 0°C.

In the present disclosure, in operation (2), the reaction temperature of the reaction is within a range of 20-30°C, preferably 22-28°C, further preferably 25°C; and the reaction time of the reaction is within a range of 2-3 h, preferably 2.5 h.

In the present disclosure, in operation (3), a mass-volume-volume ratio of the compound 1e, the sodium borohydride solution, and tetrahydrofuran is within a range of (40-45) g: (18-22) mL: (140-160) mL, preferably within a range of (41-44) g: (19-21) mL: (145-155) mL, and further preferably within a range of (42-43) g: 20mL: 150mL. A concentration of the sodium borohydride solution is within a range of 0.8-1.0 g/mL, preferably within a range of 0.85-0.95 g/mL, further preferably within a range of 0.87-0.93 g/mL.

The compound 1e is

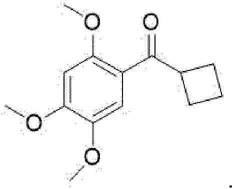

In the present disclosure, the sodium borohydride solution is mixed at a temperature within a range of -2 to 2°C, preferably 0°C.

In the present disclosure, a concentration of the sodium hydroxide solution is within a range of 8-11 wt%, preferably within a range of 9-10 wt%, and further preferably 10 wt%. The amount of the sodium hydroxide solution is preferably 10 drops.

In the present disclosure, in operation (3), a reaction temperature of the reaction is within a range of 50-65°C, preferably within a range of 55-65°C, further preferably 60°C. A reaction time of the reaction is within a range of 2-4 h, preferably within a range of 2.5-3.5 h, further preferably 3 h.

In the present disclosure, in operation (4), a mass-mass-volume ratio of the compound 1f, sodium acetate, and acetic anhydride is within a range of (38-45) g: (7-10) g: (200-230) mL, preferably within a range of (39-44) g: (7.5-9.5) g: (205-225) mL, further preferably within a range of (40-43) g: (8-9) g: (210-220) mL.

The compound 1f is

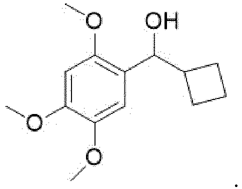

In the present disclosure, in operation (4), the reaction temperature of the reaction is within a range of 120-140°C, preferably within a range of 125-140°C, further preferably within a range of 130-140°C. The reaction time is within a range of 2-4 h, preferably within a range of 2.5-3.5 h, further preferably 3 h.

In the present disclosure, a reaction route is as follows (compound 1 is a 1-(cyclobutylidenemethyl)-2, 4, 5-trimethoxybenzene compound):

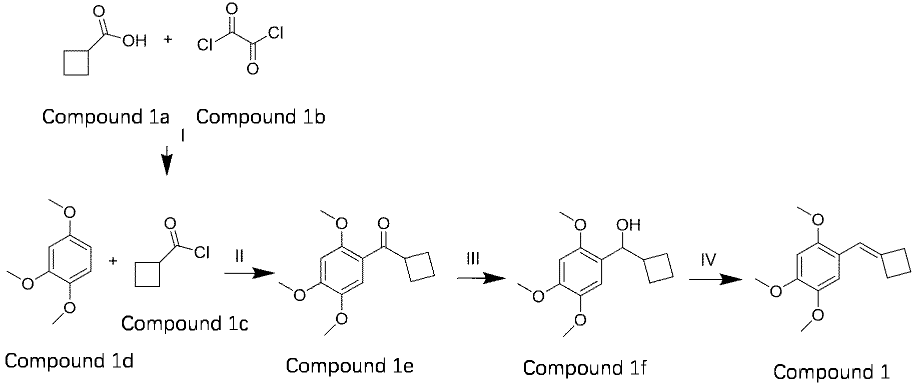

The present disclosure provides use of the 1-(cyclobutylidenemethyl)-2, 4, 5-trimethoxybenzene compound in preparation of an antiepileptic drug.

The present disclosure provides use of the 1-(cyclobutylidenemethyl)-2, 4, 5-trimethoxybenzene compound in preparation of a drug for treatment and/or prevention of traumatic craniocerebral injury disorders.

The present disclosure provides use of the 1-(cyclobutylidenemethyl)-2, 4, 5-trimethoxybenzene compound in preparation of a drug for treatment and/or prevention of ischemic stroke.

The present disclosure provides use of the 1-(cyclobutylidenemethyl)-2, 4, 5-trimethoxybenzene compound in preparation of a drug for treatment and/or prevention of hemorrhagic stroke.

The present disclosure provides use of the 1-(cyclobutylidenemethyl)-2, 4, 5-trimethoxybenzene compound in preparation of a drug for prevention and/or treatment of Parkinson's disease.

The technical solutions provided by the present disclosure are described in detail below in connection with the embodiments, but they are not to be construed as limiting the scope of protection of the present disclosure.

### Embodiment 1

25.00 g of compound 1a was dissolved in 150mL of dichloromethane under the protection of nitrogen to obtain a mixture, the mixture was stirred uniformly, then 95.08 g of compound 1b was added to the mixture drop by drop for reaction. The reaction temperature of the reaction was 25°C and the reaction was carried out under a stirring condition. The reaction was ended until no bubble was generated under the stirring condition, and 25.00 g of an orange-yellow liquid was obtained after concentration, which was compound 1c, with a yield of 84.4%.

25.00 g of compound 1c and 31.95 g of compound 1d were dissolved in 100 mL of dichloromethane to obtain a mixture, and 28.12 g of aluminum trichloride was added to the mixture when the temperature of the mixture was reduced to 0°C. Then, the reaction system was warmed up to 25°C and reacted for 2.5 h to obtain a reaction product. The reaction product was then quenched with 300 mL of water and then extracted with ethyl acetate (3×200 mL). Organic phases were combined and dried with anhydrous magnesium sulfate. After that, a crude product was obtained by concentration, and the crude product was purified by pulping with 40 mL of ethanol for 1 h to obtain 43.70 g of white solid, which was compound 1e, with a yield of 91.9%.

43.70 g of compound 1e was dissolved in 150 mL of tetrahydrofuran to obtain a mixture, and 20 mL of sodium borohydride solution was added dropwise to the mixture when the mixture was lowered to 0°C. The concentration of sodium borohydride solution was 0.925 g/mL. After that, 10 drops of 10 wt% sodium hydroxide solution were added to the mixture, then the reaction system was heated up to 60°C and reacted for 3 h. Then, the reaction system was cooled down to 37°C and then pH was adjusted to 7 with a 1M hydrochloric acid solution to obtain a reaction product. The reaction product was concentrated to remove the tetrahydrofuran and then extracted with ethyl acetate (3×200 mL). Organic phases were combined and dried with anhydrous magnesium sulfate and then concentrated to obtain 42.59 g of orange-yellow semi-solid, which was compound 1f, with a yield of 96.7%.

42.59 g of compound 1f and 8.31 g of anhydrous sodium acetate were dissolved in 210 mL of acetic anhydride to obtain a mixture, and the reaction system was warmed up to 140°C to react for 3 h to obtain a reaction product. After that, the reaction product was concentrated to remove the acetic anhydride, quenched by adding 200 mL of water, and extracted with ethyl acetate (4×200 mL). Organic phases were combined, dried with anhydrous magnesium sulfate, and concentrated to obtain a crude product, which was finally purified by recrystallization with 70% ethanol to obtain 23.47 g of 1-(cyclobutylidenemethyl)-2, 4, 5-trimethoxybenzene compound as a white solid, with a yield of 59.4%.

¹H-NMR (400MHz, CDCl₃) δ 6.82 (s,1H), 6.51 (s, 1H), 6.34 (t, J=2.4Hz, 1H), 3.88 (s, 3H), 3.82 (s, 3H), 3.81 (s, 3H), 3.13~2.95 (m, 2H), 2.89 (d, J = 6.1 Hz, 2H), 2.09 (p, J=7.8 Hz, 2H).

¹³C-NMR (100 MHz, CDCl₃) δ 150.7, 148.0,143.1, 142.5, 119.0, 114.5, 111.4, 98.0, 56.9, 56.6, 56.2, 32.8, 32.6, 18.5.

### Embodiment 2

27.00 g of compound 1a was dissolved in 170 mL of dichloromethane under nitrogen protection to obtain a mixture, the mixture was stirred uniformly, then 97 g of compound 1b was added drop by drop to carry out the reaction. The reaction temperature was 30°C, and the reaction was carried out under a stirring condition. The reaction ended until no bubble was generated under the stirring condition, and 26.62 g of an orange-yellow liquid was obtained by concentration, which was compound 1c, with a yield of 83.6%.

26.62 g of compound 1c and 33 g of compound 1d were dissolved in 120 mL of dichloromethane to obtain a mixture, and 30 g of aluminum trichloride was added to the mixture when the temperature of the mixture was reduced to 0°C. Then, the reaction system was warmed up to 30°C to react for 2 h to obtain a reaction product. The reaction product was then quenched with 300 mL of water and extracted with ethyl acetate (3 × 200 mL). Organic phases were combined, dried with anhydrous magnesium sulfate, and then concentrated to obtain a crude product. The crude product was purified by pulping with 40 mL of ethanol for 1 h to obtain 44.05 g of white solid, which was compound 1e, with a yield of 89.7%.

44.05 g of compound 1e was dissolved in 160 mL of tetrahydrofuran to obtain a mixture, and 22 mL of sodium borohydride solution was added dropwise to the mixture when the temperature of the mixture was lowered to 0°C, the concentration of sodium borohydride solution was 1.0 g/mL, followed by the addition of 10 drops of 10 wt% of sodium hydroxide solution. Then, the reaction system was heated up to 65°C to react for 2 h. Then, the reaction system was cooled down to 37°C and then pH was adjusted to 8 with a 1M hydrochloric acid solution to obtain a reaction product. After that, the reaction product was concentrated to remove the tetrahydrofuran, and then extracted with ethyl acetate (3 × 200 mL). Organic phases were combined and dried with anhydrous magnesium sulfate and then concentrated to obtain 40.10 g of orange-yellow semi-solid, which was the compound 1f, with a yield of 90.3%.

40.10 g of compound 1f and 10 g of anhydrous sodium acetate were dissolved in 230 mL of acetic anhydride, and the reaction system was warmed up to 140 ° C to react for 2 h to obtain a reaction product. The reaction product was concentrated to remove the acetic anhydride, quenched by adding 200mL of water, and extracted with ethyl acetate (4×200 mL). Organic phases were combined, dried with anhydrous magnesium sulfate, and concentrated to obtain a crude product, and the crude product was finally recrystallized and purified with 70% ethanol to obtain 22.59 g of 1-(cyclobutylidenemethyl)-2, 4, 5-trimethoxybenzene compound as white solid with a yield of 59.2%.

### Embodiment 3

23.00 g of compound 1a was dissolved in 130 mL of dichloromethane under nitrogen protection to obtain a mixture, and the mixture was stirred uniformly, then 93 g of compound 1b was added drop by drop to carry out the reaction. The reaction temperature was 20°C, and the reaction was carried out under a stirring condition. The reaction ended until no bubble was generated under the stirring condition, and 22.87 g of an orange-yellow liquid was obtained by concentration, which was compound 1c, with a yield of 84.0%.

22.87 g of compound 1c and 30 g of compound 1d were dissolved in 80 mL of dichloromethane to obtain a mixture, and 26 g of aluminum trichloride was added to the mixture when the temperature of the mixture was reduced to 0°C. Then, the reaction system was warmed up to 20°C to react for 3 h to obtain a reaction product. The reaction product was then quenched with 300 mL of water and extracted with ethyl acetate (3 × 200 mL). Organic phases were combined, dried with anhydrous magnesium sulfate, and then concentrated to obtain a crude product. The crude product was purified by pulping with 40 mL of ethanol for 1 h to obtain 40.22 g of white solid, which was compound 1e, with a yield of 90.1%.

40.22 g of compound 1e was dissolved in 140 mL of tetrahydrofuran to obtain a mixture, and 18 mL of sodium borohydride solution was added dropwise to the mixture when the temperature of the mixture was reduced to 0°C, the concentration of sodium borohydride solution was 0.8 g/mL, followed by the addition of 10 drops of 10 wt% of sodium hydroxide solution. Then, the reaction system was heated up to 50°C to react for 4 h, and the reaction system was cooled down to 37°C and then pH was adjusted to 7 with a 1M hydrochloric acid solution to obtain a reaction product. After that, the reaction product was concentrated to remove the tetrahydrofuran, and then extracted with ethyl acetate (3×200 mL). Organic phases were combined, dried with anhydrous magnesium sulfate, and then concentrated to obtain 38.31 g of orange-yellow semi-solid, which was the compound 1f, with a yield of 94.5%.

38.31 g of compound 1f and 7 g of anhydrous sodium acetate were dissolved in 200 mL of acetic anhydride, and the reaction system was warmed up to 120 ° C to react for 4 h to obtain a reaction product. The reaction product was concentrated to remove the acetic anhydride, quenched by adding 200 mL of water, and extracted with ethyl acetate (4×200 mL). Organic phases were combined, dried with anhydrous magnesium sulfate, and concentrated to obtain a crude product, and the crude product was finally recrystallized and purified with 70% ethanol to obtain 21.10 g of 1-(cyclobutylidenemethyl)-2, 4, 5-trimethoxybenzene compound as white solid, with a yield of 59.3%.

### Performance test

1-(cyclobutylidenemethyl)-2, 4, 5-trimethoxybenzene compound (compound 1, hereinafter referred to as CTMB)

Preparation process of emulsion injection was as follows. 10.0 g of CTMB obtained in embodiment 1 and 200.0 g of soybean oil for injection were weighed and placed in a beaker, then heated to 75°C under nitrogen protection and was stirred to dissolve to obtain a mixture. 12.0 g of egg yolk lecithin was then weighed and added to the mixture, then stirred to dissolve to obtain an oil phase for later use. In addition, 22.5 g of glycerol was weighed and about 680 mL of water was measured and placed in a beaker, and heated to 75°C under nitrogen protection and stirred to dissolve to obtain an aqueous phase. The oil phase was added to the aqueous phase, sheared at high speed for 10 min, and water was replenished to a total volume of 1000 mL, then colostrum was prepared. The colostrum was homogenized twice with a high-pressure homogenizer, so that the average particle size of the homogenized droplets was not greater than 0.4 µm. The pH of emulsion was adjusted to 8.5, and the emulsion was filled into 5 mL glass ampoules under nitrogen protection. The glass ampoules filled with the emulsion were subjected to the rotary hot-pressure sterilization at 121°C for 12 min, then the emulsion injection (named as CTMB emulsion injection) was obtained, and a concentration of CTMB in the emulsion injection is 10 mg/mL. Blank emulsion injection without CTMB was prepared in the same way.

Unless otherwise specified during testing, CTMB was administered as emulsion injection.
(1). Vali2dation of the protective effect of CTMB on a mouse model of pentylenetetrazole-induced epilepsy and the protective effect of CTMB on a rat model of lithium-pilocarpine-induced epilepsy

### Test 1

Validation of the protective effect of CTMB in a mouse model of pentylenetetrazole-induced epilepsy.
(1) Experimental animals: specific pathogen free (SPF) adult male Kunming (KM) mice with a weight of 30-35g.
(2) Animal grouping: the test mice were randomly divided into 3 groups, namely, saline group, low-dose CTMB group (a dose of 50 mg/kg, CTMB-L), middle-dose CTMB group (a dose of 75mg/kg, CTMB-M), high-dose CTMB group (a dose of 100 mg/kg, CTMB-H), 10 mice in each group, and each group was administered by intraperitoneal injection.
(3) Evaluation methods and results:

After a period of drug administration, mice in each group were injected intraperitoneally with pentylenetetrazole of the epilepsy modeling drug with a dose of 75 mg/kg, and clonic seizures and tonic seizures were used as evaluation indexes. Racine scores as well as the seizure rate and latency period of clonic seizures (Racine scores of 1-3) and tonic seizures (Racine scores of 4-5) were observed and recorded for each group of mice within 30 min. Grading scale of Racine scores are as follows. Grade 0 represents normal nonepileptic activity; grade 1 represents wet-dog-like shaking or scratching; grade 2 represents head nodding or tail wagging; grade 3 represents stretching of one forelimb or clonus of one limb; grade 4 represents multiple limb clonus or tonicity; grade 5 represents falls and generalized tonic clonic seizures. If no seizure occurs in the mouse within 30 min, the latency period was recorded as 1800 s. The results of the experiment were shown in Table 1:

**Table 1 Protective effect of CTMB on the mouse model of pentylenetetrazole-induced epilepsy**

| Group (ip) | Administration dose (mg/kg) | Seizure grade | seizure rate | | Incubation period (s) | |
|---|---|---|---|---|---|---|
| | | | Clonic seizure | Tonic seizure | Clonic seizure | Tonic seizure |
| Saline group | - | 5.0±0.0 | 100% | 100% | 103.6±101.6 | 152.4±160.3 |
| CTMB-L | 50 | 4.1±1.5 | 90% | 70% | 588.9±657.6** | 1000.8+700.7** * |
| CTMB-M | 75 | 1.8±1.3** | 50%* | 30%** | 106.0±789.4*** | 1553.5+400.4** * |
| CTMB-H | 100 | 0.2+0.4*** | 20%*** | 0%*** | 1549.9+537.1*** | 1800.0+0.0*** |

Compared with the saline group, *P<0.05, **P<0.01, and ***P<0.001.

As seen in Table 1, CTMB significantly prolongs the latency period of clonic and tonic seizures caused by pentylenetetrazole, and reduces the grade and incidence of seizures, and its antiepileptic effect is dose-dependent.

### Test 2

Verification of protective effect of CTMB on a rat model of lithium-pilocarpine-induced epilepsy
(1) Experimental animals: SPF adult male Sprague Dawley (SD) rats, with a weight of 200-250g.
(2) Establishment of the rat model of lithium-pilocarpine-induced epilepsy

Methylscopolamine bromide, lithium chloride, and pilocarpine were weighed, and experimental drugs were dissolved to working concentrations (100 mg/mL of lithium chloride solution, 1 mg/mLof methylscopolamine bromide solution, and 20 mg/mL of pilocarpine solution) with 0.9% saline. Rats were injected intraperitoneally with lithium chloride (a dose of 127 mg/kg), and 18-20 h later, scopolamine methylbromide (a dose of 1 mg/kg) was injected intraperitoneally. The first dose of 30 mg/kg pilocarpine intraperitoneally was injected intraperitoneally after 30 min, and the degree of epileptiform seizures was observed in rats. Thereafter, an additional dose of 10 mg/kg pilocarpine was injected intraperitoneally every 30 min until the onset of a grade IV or higher status epilepticus (SE)-like seizure without significant intermittent intervals, with a maximum dose of 60 mg/kg.

Model inclusion criterion: experimental animals presenting continuous seizures of grade IV or higher for 1 h was judged as successful modeling.

### (3) Animal grouping

The rats with successful modeling were randomly divided into three groups, namely a model group, a CTMB group, and a sodium valproate (VPA) group. Administration was carried out after 1 h of SE onset, with a blank emulsion given by intraperitoneal injection in the model group, a CTMB emulsion injection at a dose of 50 mg/kg given by intraperitoneal injection in the CTMB group, and sodium valproate at a dose of 300 mg/kg given by oral gavage in the VPA group.

### (4) Evaluation methods and results

Behavioral assessment of post-seizure seizures after pharmacological intervention

Referring to the grading scale of Racine scores, the seizure symptoms of each group of rats after the drug intervention were observed, and the seizure grades of each group of rats in the acute phase were recorded. Grading scale of Racine scores is as follows: grade 0 represents normal non-epileptic activity; grade 1 represents wet-dog-like shaking or scratching; grade 2 represents head nodding or tail wagging; grade 3 represents one forelimb extension or one limb clonic; grade 4 represents multiple limb clonic or tonic; grade 5 represents falls and generalized tonic clonic seizures; grade 6 represents seizures severe enough to kill. The results of the test were presented in Table 2.

**Table 2 Effect of CTMB on the highest seizure grade score in SE model rats**

| Group | Mold | CTMB (50 mg/kg) | VPA (300 mg/kg) |
|---|---|---|---|
| Seizure grade score | 5.44±0.53 | 4.38±0.52*** | 5.00+0.00# |

| | | | |
|---|---|---|---|
| Note: Comparison of CTMB group with model group, ***P<0.001, t-test; Comparison of CTMB group with VPA group, ^{#}P <0.05, t-test. | | | |

As shown in Table 2, CTMB (50 mg/kg) significantly reduces the seizure grade in the acute phase of the SE model and the effect of CTMB is significantly more effective than the positive drug VPA (300 mg/kg).

### Observation of one-week survival

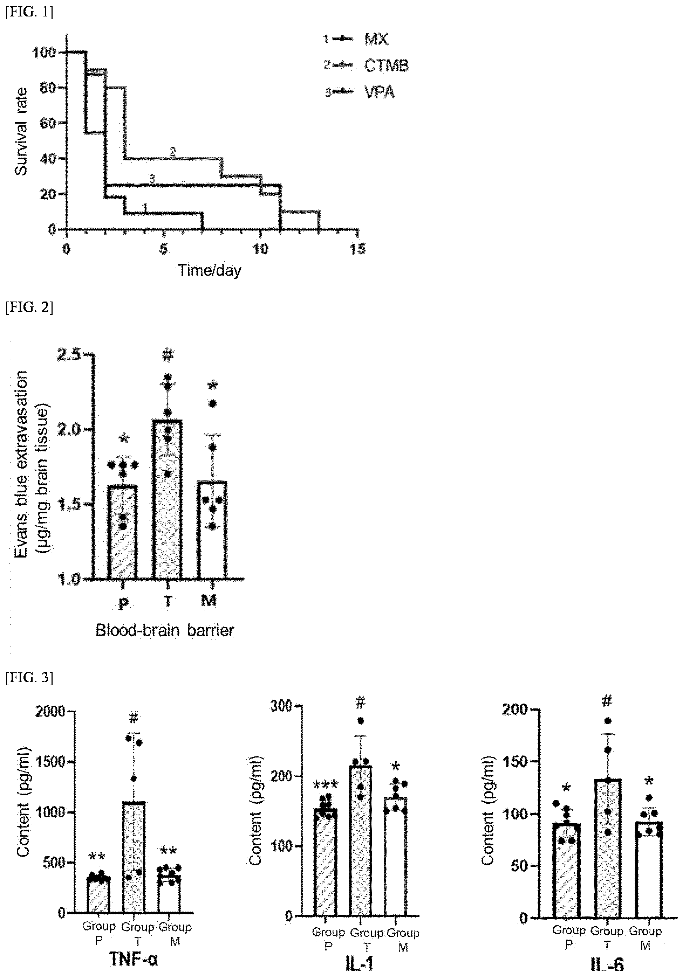

Survival of SE model rats was observed after an acute phase of drug intervention for two weeks, and the results were shown in FIG. 1.

The differences between the survival curves of the rats in each group were analyzed by a Logrank test. The results show that CTMB (50 mg/kg) significantly increases the survival rate of the SE model rats (P<0.05), and its effect is better than that of the positive drug VPA.

(2). Study on the mechanism of CTMB in the treatment of traumatic craniocerebral injury

### Laboratory animals and laboratory materials

SPF male SD rats with a weight of 240-260 g, which were purchased from Chengdu Dashuo Laboratory Animal Co., Ltd., Sichuan Province, Certificate of Conformity No.: SCXK (Sichuan) 2020-030;
Evans blue (C11891158, Shanghai Macklin Biochemical Co., Ltd.);
Formamide (20190716, Tianjin Bodi Chemical Co., Ltd.,);
IL-1 assay kit (E-EL-H0149c, Elabscience Biotechnology Co., Ltd., Wuhan);
IL-6 assay kit (E-EL-H2518c, Elabscience Biotechnology Co., Ltd., Wuhan);
TNF-α assay kit (9680019151122, ABclonal Biotech Co., Ltd);
GABA assay kit (ZD0342B47674, Elabscience Biotechnology Co., Ltd., Wuhan);
GLT-1 assay kit (Apr2023, Ruixin Biotechnology Co., Ltd., Quanzhou).

### Test 3

### Blood-brain barrier permeability test

The experimental grouping and drug administration plan were as follows.
Sham operation group (Group P): saline was given with the same volume as in the CTMB group;
Model group (T group): a blank emulsion was given with the same volume as in the CTMB group;
Medium dose group (Group M): a dose of 30 mg/kg of CTMB emulsion injection was given.

After 2 h of traumatic brain injury (TBI) modeling, the rats in each group were administered with intraperitoneal injection. After 24 h of the administration, 4% Evans blue solution (2.5 mL/kg) was injected into the right tail vein of the rats, and after 1 h, the rats were deeply anesthetized. The rats were perfused with 100 mL of saline through the heart, the brains were rapidly severed and removed and were immediately divided into the left hemisphere, the right hemisphere, the cerebellum, and the brain stem. The brain tissues were weighed to wet weight, soaked in 10 times volume of pure formamide, incubated at 60°C for 48 h, and centrifuged at 25°C and 10000 rpm/min for 30 min. The supernatant was aspirated to detect the Evans blue dye by UV spectrophotometry at 622 nm and a standard curve was plotted for quantification, and the final results were shown as Evans blue content per gram of brain tissue (µg/g). The results were shown in FIG. 2 (n=6). Compared with the sham operation group (Group P), the hemisphere on the hemorrhage side of the model group (Group T) shows increased blood-brain barrier permeability at 24 h postoperatively, while the CTMB medium dose group (Group M) significantly reduces Evans blue exudation and improves blood-brain barrier integrity.

### Test 4

### Test of IL-1, IL-6, and TNF- α

The experimental grouping and drug administration plan were the same as the test 3. After 2 h of the TBI modeling, different groups of rats were administered intraperitoneally. After 24 h of the administration, the rats were deeply anesthetized, the head was severed and the brain was removed, about 60-120 mg of hemisphere cortex was taken out from the hemisphere on the blood side, 10 times the volume of ice-cold centrifugal buffer used for biochemical assays was added, homogenization was carried out in an ice bath for 10 min, and centrifugation was carried out at 4°C and 14000 rpm/min for 30 min. The supernatant was extracted, and inflammatory factors IL-1, IL-6, and TNF-α were tested according to the instructions of IL-1, IL-6, and TNF-α assay kits, respectively. The results were shown in FIG. 3 (n=5-8). Compared with group P, the contents of IL-1, IL-6, and TNF-α in the hemorrhagic lateral cerebral hemisphere of rats in group T are significantly increased at 24 h postoperatively, whereas the contents of pro-inflammatory cytokine TNF-α and inflammatory factors IL-1 and IL-6 in the cerebral cortex tissues of TBI rats in group M are significantly reduced, which is conducive to the alleviation of neuroinflammation and improvement of the motor function of the rats.

### Test 5

### Determination of GLT-1 and GABA content

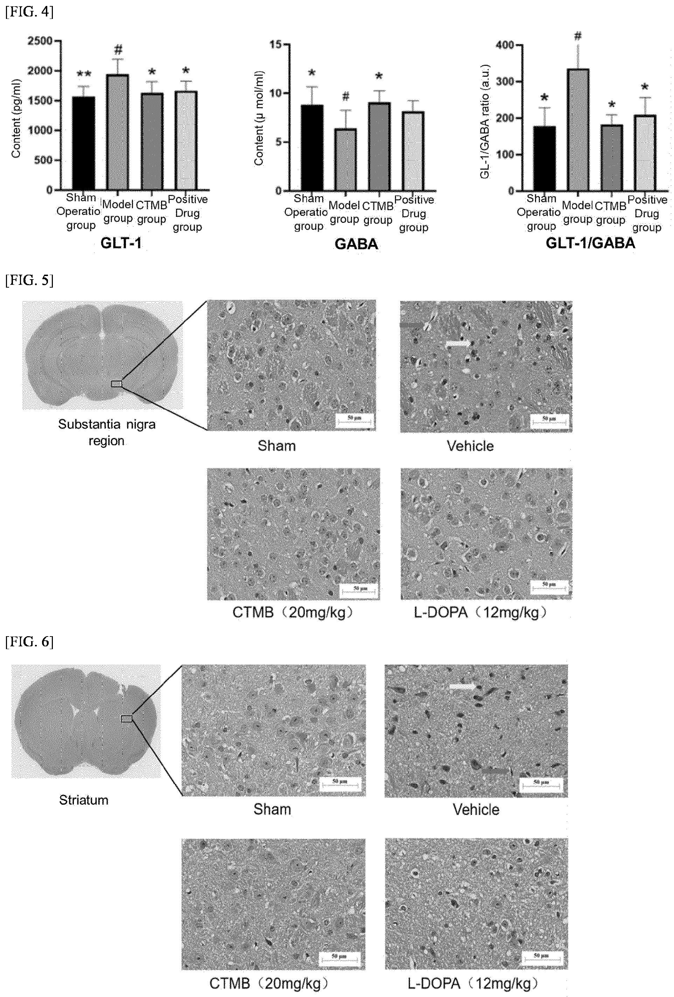

The experimental grouping and drug administration plan were the same as the test 3. After 7 d of continuous administration to each group, the rats were deeply anesthetized, the heads were severed and the brains were removed, about 60-120 mg of hemispheric cortex on the blood side was taken out, 10 times volume of ice-cold centrifugation buffer used for biochemical assay was added, homogenized for 10 min under an ice bath, and then centrifuged for 30 min at 4°C and 14000 rpm/min. The supernatants were taken to test the GLT-1 and GABA content according to the instructions of GLT-1 and GABA content assay kit, respectively. Glutamate transporter 1 (GLT-1) is an important glutamate transporter in the brain that transports extracellular glutamate to the intracellular compartment. γ -aminobutyric acid (GABA) is an important inhibitory neurotransmitter, and a dramatic increase in the concentration of extracellular glutamate in pathological conditions leads to neuronal hyperexcitability toxicity. FIG. 4 shows the contents of GLT-1 and GABA measured in different drug administration groups and the ratio of the two, which may be used to assess the excitatory neurotoxicity effect caused by TBI. The results were shown in FIG. 4 (n=6), compared with the group P, the GLT-1 content and the ratio of GLT-1 and GABA in the hemorrhagic lateral cerebral hemisphere of rats in group T are significantly increased after 7 days postoperatively, whereas the administration of group M reduces the GLT-1 content in brains of the TBI rats, increases the content of GABA, and significantly reduces the ratio of GLT-1 and GABA, so as to restore the cerebral excitatory amino acid (EAA) and inhibitory amino acid (IAA) balance in the brain, reduce the excitatory neurotoxicity caused by excessive glutamate, and then improve the motor function of rats.

(3). Validation of the therapeutic effect of CTMB on the acute phase of ischemic stroke induced by the filament technique in rats
3.1 Experimental materials: SPF male SD rats with a weight of 200-230 g (purchased from Sichuan Dashuo Laboratory Animal Co., Ltd., Certificate of Conformity No.: SCXK (Chuan) 2020-0030);
MCAO filament (purchased from Beijing Cinotech Co., Ltd., model: 2432-A5);
Edaravone dextran campesterol concentrated solution for injection (purchased from Simcere Pharmaceutical Co., Ltd., Specification: edaravone 10 mg/5mL, exborneol 2.5 mg/mL; Lot No.: 180-220522).

3.2 Experimental grouping: Zea Longa score was used to determine whether the modeling was successful after 2 h of MCAO surgery, and rats with successful modeling were taken and randomly grouped for administering drugs.

The rats were randomly divided into a sham operation group (Sham group, given a blank emulsion with a volume equal to that of the high-dose CTMB group), a model group (Vehicle group, given a blank emulsion with a volume equal to that of the high-dose CTMB group), a low-dose CTMB group (CTMB-L group, 10 mg/kg), a high-dose CTMB group (CTMB-H group, 20 mg/kg), and an edaravone dexborneol injection concentrated solution group (EDB group, commercially available, 3 mg/kg). 10 rats were included in each group, which were administered by intraperitoneal injection. It should be noted that in the experiment, the mode of administration for rats was intraperitoneal injection, and a ratio of the effective dose of CTMB, the active ingredient in the emulsion injection, to the unit mass of the human body was within a range of 0.2 mg-4.0 mg/kg, which referred to the ratio of the effective dose of CTMB, the active ingredient in the emulsion injection, to the unit mass of the human body during intravenous infusion, which was extrapolated from the effective dose of the rats. Considering the different bioavailability, peak concentration, and peak time of the drug due to different species and different modes of administration, the effective dose of the active ingredient CTMB in the emulsion injection to the unit mass of human body was finally determined to be 0.2 mg-4.0 mg/kg through conversion.

### 3.3 Ischemia-reperfusion modeling by the filament technique

The rats were fasted for 12 h before procedure, and the rats were anesthetized with 10% chloral hydrate (350 mg/kg, i.p.). The rats were fixed in the supine position, and the body temperature was maintained at about 37°C. For neck preparation, a median neck incision was made, the muscle and fascia were separated along the inner edge of the sternocleidomastoid muscle to expose the right side, the common carotid artery (CCA), external carotid artery (ECA), and internal carotid artery (ICA) were separated bluntly, and the lines were disposed of proximally in the CCA proximal end, ICA, and ECA for backup. The CCA proximal end and ECA were ligatured, and an arterial clip was used to temporarily close the ICA, then a small hole was poked at a position of CCA having about 4 mm from the bifurcation with a needle. The filament was inserted into the ICA through the CCA, the arterial clipped on ICA was loosened, and the filament was slowly advanced. When the mark on the filament reached the bifurcation and a slight resistance was felt, the thin wire at ICA was tightened, and saline was used to clean the wound and then suture was performed. The sham procedure was the same as the experimental group except that no filament was inserted. After anesthesia, the rats were raised normally.

### 3.4 Inclusion criterion of cerebral ischemia model

Referring to the Zea Longa Neurologic Function Score (Longa EZ, Weinstein PR, Carlson S, Cummins R. Reversible middle cerebral artery occlusion without craniectomy in rats. Stroke. 1989 Jan; 20(1):84-91. doi: 10.1161/01.str.20.1.84.), rats were scored after 2 h of procedure when they were awake from anesthesia. Those with a score of 1-2 were enrolled in the group. The score 0 represents no signs of neurological deficit and normal activity; 1 represents the inability to fully extend the contralateral forepaw; 2 represents spinning when the animal was crawling; 3 represents body tilted to the hemiplegic side tilt; 4 represents the inability to walk spontaneously and lost consciousness.

### 3.5 Short-term neurological deficit score

The neural function of the rats was comprehensively evaluated after 24 h of modeling using the modified mNSS score and Garcia score, and mNSS scoring criteria was shown in Table 3 and Garcia scoring criteria was shown in Table 4. The rats were evaluated for locomotion, sensation, and climbing, and symmetry of limbs. The mNSS score was within a range of 0-18 points, and the higher the score, the worse the neurological function damage. The Garcia score was within a range of 3-18 points, and the lower the score, the worse the neurological function damage. Scoring was done independently by uninformed persons who were not involved in modeling and drug administration.

**Table 3 mNSS neurological function score**

| | Observation project | Score |
|---|---|---|
| Tail lifting test (3 points) | Forelimb flexion | 1 point |
| | Hindlimb flexion | 1 point |
| | An angle of head deviating from a vertical axis more than 10° within 30 s | 1 point |
| Walking test (3 points) | Normal walking | 0 point |
| | Inability in normal walking | 1 point |
| | Spin toward the paraplegic side | 2 points |
| | Tipping to the paraplegic side | 3 points |
| Sensory test (2 points) | Placement test (visual and tactile tests) | 1 point |
| | Proprioceptive test (stimulation of limb muscles by pressing the rat's paw towards the edge of the table) | 1 point |
| Balance Beam test (6 points) | Stabilizing balance posture | 0 point |
| | Holding on to the edge of the balance beam | 1 point |
| | Clutching the balance beam, slipping on one limb | 2 points |
| | Clutching the balance beam, dipping or rotating two limbs from the beam (more than 60 s) | 3 points |
| | Attempting to balance on the balance beam but fell (more than 40 s) | 4 points |
| | Attempting to balance on the balance beam but fell (more than 20 s) | 5 points |
| | Falling without attempting to balance on the balance beam (more than 20 s) | 6 points |
| Reflexes and unusual activity (4 points) | Auricular reflex (head shaking on contact with external ear canal) | 1 point |
| | Corneal reflex (blinking when the cornea is touched lightly with a cotton swab) | 1 point |
| | Panic reflex (motor reflex or screaming after hearing a sudden sound) | 1 point |
| | Epilepsy, myoclonus, dystonia | 1 point |

**Table 4 Garcia neurological function score**

| | Observation project | Score |
|---|---|---|
| Spontaneous movement (place rats in a rat cage and observe for 3 min) | NO autonomic movement | 0 point |
| | Very little movement | 1 point |
| | Move and touch at least one side of the cage wall | 2 points |
| | Move and touch at least three sides of the cage wall | 3 points |
| Body symmetry (Lift the tail to suspend it and observe the state of the limbs) | No movement on the affected side | 0 point |
| | Slight movement on the affected side | 1 point |
| | Slower movement on the affected side | 2 points |
| | Bilateral body symmetry | 3 points |
| Front limb extension (Suspend the tail so that the hind limbs hang in the air and the forelimbs are moved towards the table so that they walk on the forelimbs only, observe forelimb movements) | No extension of the left forelimb | 0 point |
| | Slight extension of the left forelimb | 1 point |
| | The left forelimb is extended but not as much as the right | 2 points |
| | Bilateral stretching symmetry | 3 points |
| Climbing test | Inability in climbing up | 1 point |
| | Left side disadvantage | 2 points |
| | Normal Climbing | 3 points |
| Bilateral body touch reflex | Left unresponsive | 1 point |
| | Left side is weaker than right side | 2 points |
| | Equal reactivity on both sides | 3 points |
| Bilateral whisker touch reflex | Left unresponsive | 1 point |
| | Left side is weaker than right side | 2 points |
| | Equal reactivity on both sides | 3 points |

As shown in Table 5, compared with the sham operation group (Sham group), the model group (Vehicle group) shows a higher mNSS score (P<0.01) and a lower Garcia score (P<0.01) after 24 h of procedure, which indicates that significant neurological deficits occurre in the model group of rats after at 24 h of MCAO procedure. The administration of different doses of CTMB (CTMB-L group, CTMB-H group) and edaravone dexborneol injection concentrated solution (EDB group) can reduce the mNSS score and increase the Garcia score to varying degrees, thereby improving the neurological deficits induced by MCAO. The CTMB-H group shows the most significant improvement in the effect of the administered drugs (P<0.01), and the efficacy of the drugs is better than that of edaravone dexborneol of the clinically used drug for the improvement of ischemic stroke. No toxic side effects associated with CTMB administration were observed during the test.

**Table 5 Short-term neurological deficit score in rats**

| Group | Neurological function score | |
|---|---|---|
| | mNSS score | Garcia score |
| Sham | 0.00±0.00** | 18.00±0.00** |
| Vehicle | 10.35±1.73^{##} | 8.55±0.68^{##} |
| CTMB-L | 6.88±2.26^{##, **} | 9.65±1.02^{##,*} |
| CTMB-H | 6.65±2.30^{##, **} | 10.05±1.81^{##, **} |
| EDB | 6.90±2.60^{##,} * | 10.00±1.86^{##, *} |

| | | |
|---|---|---|
| Note: compared to the sham operation group (Sham group), ^{##}P<0.01; compared to model group (Vehicle group), **P<0.01, *P<0.05. | | |

(4) Long-term therapeutic effect of CTMB on ischemic stroke induced by the filament technique in rats.

### 4.1 Neurological deficit score

Experimental materials, groups (n=10-15), modeling manner, and scoring criteria were the same as the acute phase of treatment. After 2 h of modeling by the filament technique, the drugs were immediately administered according to the grouped administration plan, and thereafter the drugs continued to be administered once a day for 14 days, and modified mNSS score was performed on days 1, 4, 7, and 14.

As shown in Table 6, compared with the sham operation group (Sham group), the mNSS score of the model group (the Vehicle group) is significantly increased on day 14 after procedure (P<0.01), which indicates that significant neurological deficits occur in the model group of rats on day 14 after MCAO procedure. Both the CTMB administration (the CTMB-H group) and edaravone dexborneol injection concentrated solution group (the EDB group) may reduce the mNSS score and improve the neurological deficits induced by MCAO to different degrees on the days 1-7. The effect of the administration of the CTMB-H group is the most significant, and the therapeutic effect is slightly better than that of edaravone dexborneol injection concentrated solution, which is a clinically used drug to improve the ischemic stroke. In addition, on day 14, the CTMB-H group is still able to significantly reduce the mNSS score. No toxic side effects associated with CTMB administration are observed during the test.

**Table 6 Long-term neurological deficit score in rats**

| Group | Score | | | |
|---|---|---|---|---|
| | Day 1 | Day 4 | Day 7 | Day 14 |
| Sham | 0.00±0.00** | 0.00±0.00** | 0.00±0.00** | 0.00±0.00** |
| Vehicle | 8.40±1.77^{##} | 5.71±0.93^{##, **} | 4.18±0.84^{##} | 4.05±0.52^{##} |
| CTMB-L | 6.45±2.37^{##,} * | 3.65±1.29^{##, **} | 3.38±0.83^{##} | 2.94±1.56^{##} |
| CTMB-H | 5.69±2.13^{##, **} | 3.75±1.67^{##. **} | 2.83±1.17^{##, *} | 2.45±1.36^{##, *} |
| EDB | 6.71±1.94^{##, *} | 3.92±1.45^{##, **} | 3.30±0.71^{##, *} | 3.13±0.98^{##} |

| | | | | |
|---|---|---|---|---|
| Note: compared with the sham operation group (Sham group), ^{##}P<0.01; compared with the model group (Vehicle group), **P<0.01, *P<0.05. | | | | |

### 4.2 Bonding test

Rats were trained in adhesion experiments for one week before modeling, and those that could successfully tear off the tape within 10 s were screened for modeling. After 2 h of modeling by the filament technique, the rats were immediately administered with the drug according to the grouped administration plan. Thereafter, the rats were continued to be administered with the drug once a day for 14 days, and adhesion experiments were performed on days 1, 4, 7, 10, and 14 to assess sensory and motor nerve function. The specific operation was as follows. A circular tape with a diameter of about 12 mm was adhered to the plantar surface of the left forelimb of the rat, and the sensation and removal time of the tape was recorded. If the mice were unable to perceive and/or remove the tape within 60 s, 60 s were recorded.

As shown in Table 7, compared with the sham operation group (Sham group), the model group (Vehicle group) has a significantly longer time to perceive the tape on day 14 after the procedure (P<0.05), which indicates that the left forelimb perceptual ability of the model group of rats is weakened on day 14 after the MCAO procedure. Different doses of CTMB and EDB can promote the restoration of the perceptual ability of the rats.

**Table 7 Time for rats to perceive tape**

| Group | Time (s) | | | | |
|---|---|---|---|---|---|
| | Day 1 | Day 4 | Day 7 | Day 10 | Day 14 |
| Sham | 6.20+5.75** | 3.30±3.06^{**} | 5.11±10.14^{**} | 3.38±4.10^{**} | 3.38±3.78^{*} |
| Vehicle | 44.33±24.09^{##} | 39.17±23.83^{##} | 41.92±25.38^{##} | 25.33±20.15^{##} | 17.17±17.08 |
| CTMB-L | 42.25±25.53^{##} | 27.50±28.05^{##} | 28.20±28.97^{#} | 27.11±30.06^{#} | 10.44±12.44 |
| CTMB-H | 31.17±24.57^{#} | 25.33±23.41^{#} | 20.50±22.99^{#} | 7.00±4.56^{#} | 8.44±11.94 |
| EDB | 36.25±23.11^{##} | 50.00±18.28^{##} | 29.90±26.31^{##} | 22.10±20.69^{#} | 17.70±19.56^{#} |

| | | | | | |
|---|---|---|---|---|---|
| Note: compared with the sham operation group (Sham group), ^{##}P<0.01 and ^{#}P<0.05; compared with the model group (Vehicle group), **P<0.01 and *P<0.05. | | | | | |

As can be seen from Table 8, compared with the sham operation group (Sham group), the time of removing the tape of model group (Vehicle group) significantly increases on day 14 after the procedure (P<0.01), which indicates that the left forelimb locomotor ability of the model group of rats is weakened on day 14 after the MCAO procedure. The CTMB-L group may significantly reduce the time of removing the tape of the rats from day 4 (P<0.05), and the CTMB-H group significantly reduces the time of removing the tape from day 10 (P<0.05), and the efficacy of the drug is better than that of edaravone dexborneol, which is a drug clinically used to improve ischemic stroke. No toxic side effects associated with CTMB administration are observed during the test.

**Table 8 Time of removing tape in rats**

| Group | Time (s) | | | | |
|---|---|---|---|---|---|
| | Day 1 | Day 4 | Day 7 | Day 10 | Day 14 |
| Sham | 12.60±7.75^{**} | 12.70±7.59^{**} | 16.67±17.65^{**} | 14.13±8.29^{**} | 13.00±9.41^{**} |
| Vehicle | 55.33±15.85^{##} | 56.17±11.26^{##} | 56.25±8.88^{##} | 50.92±14.57^{##} | 46.67±20.02^{##} |
| CTMB-L | 50.75±17.65^{##} | 36.20±24.17^{*} | 38.60±26.15^{#,} * | 26.63+27.80^{*} | 26.22+21.48* |
| CTMB-H | 53.67±16.66^{##} | 51.33±17.14^{##} | 48.67±16.55^{##} | 31.11±19.76^{*} | 33.22±23.03^{#} |
| EDB | 52.36±13.88^{##} | 57.36±8.74^{##} | 42.70±20.78^{#} | 43.70±16.95^{##} | 43.30±19.55^{##} |

| | | | | | |
|---|---|---|---|---|---|
| Note: Compared with sham operation group (Sham group), ^{##}P<0.01 and ^{#}P<0.05; compared with model group (Vehicle group), **P<0.01 and *P<0.05. | | | | | |

In summary, long-term administration of CTMB significantly promotes the recovery of motor function and significantly improves limb perception in rats.

(5). Validation of the therapeutic effect of CTMB on acute phase of hemorrhagic stroke induced by caudate nucleus collagenase VII injection in rats
5.1: Experimental materials: SPF male SD rats with a weight of 220-250 g (purchased from Sichuan Dashuo Laboratory Animal Co., Ltd., Certificate of Conformity No.: SCXK (Chuan) 2020-0030);
collagenase VII (purchased from Sigma-Aldrich, USA, Specification: 1.5KU; Batch No.: 0000111586);
Edaravone dexborneol injection concentrated solution (purchased from Simcere Pharmaceutical Co., Ltd., Specification: edaravone 10 mg/5mL, dexborneol 2.5 mg/mL; Lot No.: 181-230207).

5.2: Experimental grouping: after 2 h of hemorrhagic stroke, Zea Longa score was used to determine whether the modeling was successful, and rats with successful modeling were taken and randomly grouped to be administered.

The rats were randomly divided into a sham operation group (Sham group, given a blank emulsion with a volume equal to that of the high-dose CTMB group), a model group (Vehicle group, given a blank emulsion with a volume equal to that of the high-dose CTMB group), a low-dose CTMB group (CTMB-L group, 10 mg/kg), a high-dose CTMB group (CTMB-H group, 20 mg/kg), and an edaravone dexborneol injection concentrated solution group (EDB group, commercially available, 3 mg/kg). Each group included 10-13 rats, which were administered by intraperitoneal injection. It should be noted that in the experiment, the mode of administration for rats was intraperitoneal injection, and a ratio of the effective dose of CTMB, the active ingredient in the emulsion injection, to the unit mass of the human body was within a range of 0.2 mg-4.0 mg/kg, which referred to the ratio of the effective dose of CTMB, the active ingredient in the emulsion injection, to the unit mass of the human body during intravenous infusion, which was extrapolated from effective dose of the rats. Considering the different bioavailability, peak concentration, and peak time of the drug due to different species and different modes of administration, the ration of the effective dose of the active ingredient CTMB in the emulsion injection to unit mass of human body was finally determined to be 0.2 mg-4.0 mg/kg through conversion.

### 5.3 Establishment of hemorrhagic stroke model by caudate nucleus collagenase VII injection

The rats were fasted and dehydrated for 8 h before the procedure, and anesthesia was induced using 4% isoflurane, and 2% isoflurane was given to maintain anesthesia, and the rats were maintained at a temperature of about 37°C. The rats were fixed at the prone position on the stereotaxic apparatus, the head was prepared for skinning, and a longitudinal incision was made in the median sagittal shape of the head to incise the skin and subcutaneous tissues, exposing the anterior chimney and coronal suture. The stereotaxic apparatus was used to locate the right caudate nucleus of the rat with the fontanel as the origin and the right paracentral opening of the midline of 3.0 mm, marked and then holes were punched with the cranial bone drill, the dura was exposed, and the hemorrhage was stopped adequately. The micro-syringe was pre-mounted on the stereotaxic apparatus, and the needle was vertically injected 5.5 mm along the cranial borehole (i.e., the caudate nucleus site). Collagenase VII (1 µ L) at a concentration of 0.5 U/µL was injected slowly and uniformly into the brain for 5 min, the needle was stopped for 8 min, and the syringe was slowly pushed out. The cranial defect was closed using sterile bone wax and the scalp incision was sutured. After disinfecting the treatment with iodophor, the rats were returned to the cage. The sham operation group (Sham group) remained unchanged except for the insertion of a needle for saline injection. After awaking from anesthesia, the rats were fed normally.

### 5.4 inclusion criteria of cerebral hemorrhage model

Referring to the Zea Longa neurologic function score (Longa EZ, Weinstein PR, Carlson S, Cummins R. Reversible middle cerebral artery occlusion without craniectomy in rats. Stroke. 1989 Jan; 20(1):84-91. doi: 10.1161/01.str.20.1.84.), the score was performed after 2 h of procedure, and those with a score of 1-2 were enrolled. 0 represents no signs of neurological deficit and normal activity; 1 represents the inability to fully extend the contralateral forepaw; 2 represents spinning when the animal was crawling; 3 represents body tilted to the hemiplegic side tilt; 4 represents the inability to walk spontaneously and lost consciousness.

### 5.5 Short-term neurological deficit score

The neural function of the rats was comprehensively evaluated after 24 h of modeling using the modified mNSS score and Garcia score, and mNSS scoring criteria was shown in Table 3 and Garcia scoring criteria was shown in Table 4. The rats were evaluated for locomotion, sensation, and climbing, and symmetry of limbs. The mNSS score was within a range of 0-18 points, and the higher the score, the worse the neurological function damage. The Garcia score was within a range of 3-18 points, and the lower the score, the worse the neurological function damage. Scoring was done independently by uninformed persons who were not involved in modeling and drug administration.

**Table 9 Short-term neurological deficit score in rats**

| Group | Neurological function score | |
|---|---|---|
| | mNSS score | Garcia score |
| Sham | 1.05±0.98^{****} | 17.20±0.48^{****} |
| Vehicle | 8.60±1.90^{####} | 8.00±1.79^{####} |
| CTMB-L | 4.54±1.81^{###, ****} | 12.45±1.89^{####, ****} |
| CTMB-H | 5.97±1.68^{####,} * | 10.45±2.49^{####, *} |
| EDB | 6.79±2.40^{####} | 10.68±3.02^{####,} * |

| | | |
|---|---|---|
| Note: compared with sham operation group (Sham group), ^{####}P<0.0001 and ^{###}P<0.001; compared with model group (Vehicle group), ****P<0.0001 and *P<0.05. | | |

As shown in Table 9, compared with the sham operation group (Sham group), the model group (the Vehicle group) shows a significantly higher mNSS score (P<0.0001) and a lower Garcia score (P<0.0001) at 24 h postoperatively, which indicates that significant neurological deficits occur in the model group of rats at 24 h postoperatively after cerebral hemorrhage. The administration of different doses of CTMB (the CTMB- L group and the CTMB-H group) can reduce the mNSS score and increase the Garcia score to different degrees, while edaravone dexborneol injection concentrated solution administration (the EDB group) can only increase Garcia score, and the CTMB administration can improve neurological deficits caused by hemorrhagic stroke. The CTMB-L group shows the most significant improvement in the effect of the administered drugs (P<0.0001), and the efficacy of the drugs is better than that of edaravone dexborneol of the clinically used drug for the improvement of ischemic stroke. No toxic side effects associated with CTMB administration is observed during the test.

### (6). Therapeutic effects of CTMB in Parkinson's disease (PD) mice

6.1 Experimental materials: SPF male C57 mice with a weight 18-22g (purchased from Chengdu Dashuo Laboratory Animal Co. in Sichuan Province, Certificate of Conformity No.: SCXK (Chuan) 2020-030), Levodopa API (Shanghai McLean Biochemical Technology Co., Ltd (Specification: 5g; Batch No.: C14100283)),

Benserazide Hydrochloride API (Shanghai Macklin Biochemical Co., Ltd., Specification: 1 g; Batch No.: C15659767).

6.2 Experimental grouping: after 2 weeks of the injection of 6-OHDA, it was determined whether the modeling was successful by using scores of the rod-climbing experiment, hanging experiment, rod-turning experiment, and the open-field experimental, and the mice that were successful in modeling were randomly divided into groups to be administered with the drugs in randomized groups.

The mice were randomly divided into a sham operation group (group S, given a blank emulsion with a volume equal to that of the administered group), a model group (group M, given a blank emulsion with a volume equal to that of the administered group), a CTMB group (20 mg/kg, group D), and a levodopa group (levodopa:benserazide hydrochloride=4:1, 12 mg/kg, group L), each group included 10-12 mice, and all of them were administered intraperitoneally.

### 6.3 Establishment of PD model by the 6-OHDA method

Preoperatively, mice were fasted for 12 h, and mice were anesthetized with 4% isoflurane induction and maintained with 1.5% isoflurane. The mice were subsequently immobilized on a brain stereotaxic apparatus. The bregma and lambda points were exposed by gently wiping the cranial surface with a cotton swab after cutting the scalp. The stereotaxic apparatus was adjusted to align the heights of the bregma and lambda points, thereby leveling the skull. The bregma point was designated as the origin, the coordinates where the injection site was located were located, and the drill was used to drill a small hole in the skull above the injection site.

6-OHDA (12.5 µg/ µL) was injected into the striatum region (AP+0.5 mm; ML+2.00 mm; DV-3.00 mm and -2.00 mm) of the mice, and 1 µL 6-OHDA was injected into each of the two points at a rate of 0.5 µL/min. The needle was left in place for 3 min after the injection was completed, and then the injection needle was lifted, and the scalp of the mice was sutured and disinfected. The sham operation group was injected with an equal volume of saline containing 0.02% ascorbic acid, and other operations were performed as above. After the procedure, the mice were placed in the cage at an appropriate temperature. After awakening from anesthesia, the mice were kept normally.

### 6.4 inclusion criteria of Parkinson's disease model

After 2 weeks of the operation, the mice were evaluated for motor function by rod-climbing experiment and hanging experiment, and those having significantly different from the sham operation group were enrolled.

The rod-climbing experiment: the rod-climbing experiment was mainly used to test the limb movement ability of mice and to assess the symptoms of motor delay. This experiment evaluated the ability of mice to grasp and maneuver on a pole to descend to their home cage. A cork ball was fixed to an iron pole with a diameter of 1cm and a length of 65 cm. The head of the mice was put down on the top of the pole, and the mice usually naturally descended down along the pole to return to their cage. The time for the mice to descend to the bottom was recorded. Results were averaged over 3 consecutive measurements.

The hanging experiment: normal mice have climbing activity, which requires proper grip strength and motor coordination, so the hanging experiment may be used to examine motor dysfunction in mice. A metal rod with a diameter of 1.5 mm was placed horizontally on top of the box, and a distance from the metal rod to the bottom of the box was 30 cm. In the experiment, the mouse 's front paws were suspended from the metal rod, and the time before landing was recorded, when the mice did not fall after 60 s, it was recorded as 60 s. Measurement was performed twice, with an interval of 1 min or more between each test, and the results were averaged over the two times.

### 6.5 Evaluation of motor function

The motor function of the mice was comprehensively assessed using the rod-climbing experiment and the hanging experiment at 1, 2, 3, and 4 weeks after drug administration. Scoring was done independently by uninformed individuals who did not participate in the modeling and drug administration.

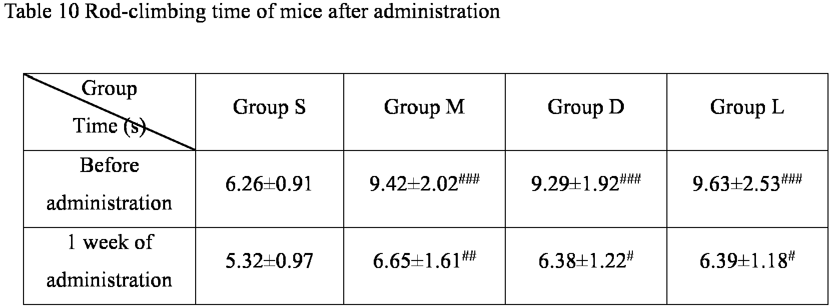

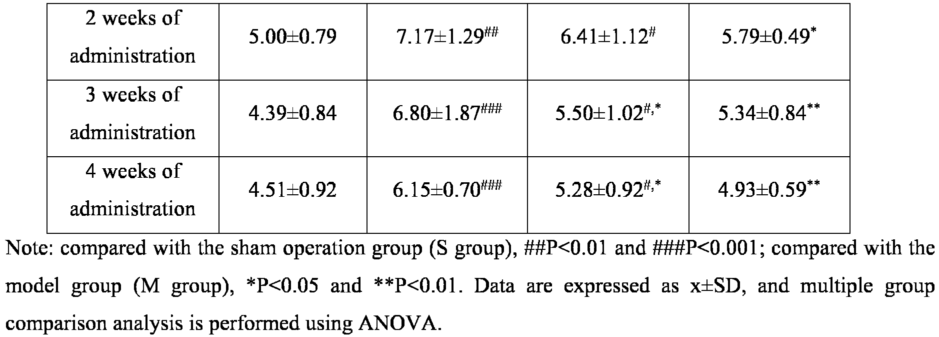

As shown in Table 10, the rod-climbing time before drug administration (at 2 weeks after procedure) is significantly higher (P<0.001) in the model group (group M) compared with the sham operation group (group S), which indicates that significant motor retardation occurs in the mice of the model group at 2 weeks after modeling. Intraperitoneal administration of CTMB group (group D) and levodopa group (group L) significantly shortens the rod-climbing time and improves 6-OHDA-induced locomotor retardation in model mice. For the PD model, the efficacy of the medication used in group D is not significantly different from that of the levodopa group (group L), which is a drug used clinically to improve Parkinson's disease. In addition, there is no significant difference in the initial rod-climbing time of all groups of mice, which may exclude the difference in behavioral function due to the difference in the degree of modeling.

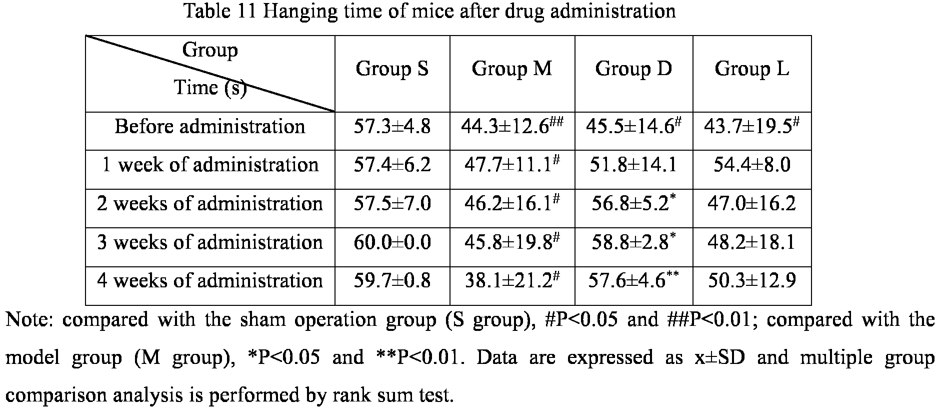

As shown in Table 11, compared with the sham operation group (Group S), the hanging time of the model group (Group M) is significantly shorter (P<0.01) before the drug administration (2 weeks postoperatively), which indicates that significant motor dysfunction occurs in the mice of the model group at 2 weeks after modeling. Intraperitoneal administration of CTMB group (group D) prolongs the hanging time and improves 6-OHDA-induced motor dysfunction in model mice. For the PD model, the efficacy of the medication used in group D is superior to that of the levodopa group (group L), which was the drug used clinically to improve Parkinson's disease, but there is no significant difference. In addition, there is no significant difference in the initial hanging time of all groups of mice, which may exclude differences in behavioral functioning due to differences in the degree of modeling.

### (7). Study on the mechanism of CTMB against Parkinson's disease

### 7.1 Experimental materials:

SPF male C57 mice with a weight of 18-22 g (purchased from Chengdu Dashuo Laboratory Animal Co. in Sichuan Province, Certificate of Conformity No.: SCXK (Chuan) 2020-030);
Hematoxylin-eosin staining solution (BP0211, Biossci (Hubei) Biotechnologies Co., Ltd.);
Toluidine blue staining solution (BP0360, Biossci (Hubei) Biotechnologies Co., Ltd.);
Recombinant Anti-Tyrosine Hydroxylase Antibody (ab137869, Abcam (Shanghai) Trading Co.),
Malondialdehyde (MDA) colorimetric test kit (TBA technology) (E-BC-K025-M, Elabscience Biotechnology Co., Ltd., Wuhan),
Total superoxide dismutase (T-SOD) colorimetric test kit (WST-1 method) (E-BC-K020-M, Elabscience Biotechnology Co., Ltd., Wuhan),
Reduced glutathione (GSH) colorimetric assey kit (E-BC-K030-M, Elabscience Biotechnology Co., Ltd., Wuhan).

### 7.2 Experimental operations and results

### 7.2.1 Sample preparation

The mice were randomly divided into a sham operation group (group S, given a blank emulsion with a volume equal to that of the administered group), a model group (group M, given a blank emulsion with a volume equal to that of the administered group), a CTMB group (20 mg/kg, group D), and a levodopa group (levodopa:benserazide hydrochloride=4:1, 12 mg/kg, group L), each group included 10-12 mice, and all of them were administered intraperitoneally.

After completing the evaluation of motor function after 4 weeks of drug administration, the mice were deeply anesthetized, perfused with 20 mL of saline and 20 mL of 4% paraformaldehyde via the heart, and the brains were rapidly severed and extracted from the head, which were then preserved and fixed in 4% paraformaldehyde for 72 h after removal, and then embedded with paraffin to prepare the slices with a thickness of 4 µm for subsequent pathological and histological studies.

7.2.2 Attenuating neuronal damage in the substantia nigra region and striatum of the midbrain of PD mice

Hematoxylin-eosin (HE) staining may be used to observe changes in the pathological condition of brain tissue. Specific operations of HE staining were as follows.
a) De-paraffinization of paraffin sections to water: paraffin slice was dewaxed sequentially in an environmental protection dewaxing agent (1), an environmental protection dewaxing agent (2), and an environmental protection dewaxing agent (3) for 10 min, and treated by anhydrous ethanol, 95% ethanol, 85% ethanol, 75% ethanol for 5 min, in sequence, then rinsed by tap water for 1 min.
b) Hematoxylin staining solution (Harris) was used to stain the slice for 4 min and washed in tap water for 2 min until no excess stain was removed from the slice.
c) The slice was subjected to alcohol differentiation with 0.8% hydrochloric acid for 2 s and rinsed by tap water, or returned to blue with aqueous lithium carbonate and washed by tap water for 2 min.
d) The slice was put into eosin staining solution (alcohol soluble) for 20 s without washing, and the slice was directly put into 95% ethanol to adjust the color for 5 s, and put into anhydrous ethanol (1), anhydrous ethanol (2) for dehydration for 2 min to obtain a product.
e) The product was cleared with eco-friendly clearing agents, mounted, and examined by microscopic.

Niehl's staining was used to identify Niehl's bodies in neuronal cells. The specific operation for Niehl' s staining was as follows.
a) De-paraffinization of Paraffin Sections to Water: paraffin slice was dewaxed sequentially in an environmental protection dewaxing agent (1), an environmental protection dewaxing agent (2), an environmental protection dewaxing agent (3) for 10 min, and then treated by anhydrous ethanol, 95% ethanol, 85% ethanol, 75% ethanol for 5 min respectively, and washed by distilled water 3 times.
(b) The slice was placed in pre-warmed 1% aqueous toluidine blue solution at 50°C and stained for 20 min at 56°C in a temperature chamber and washed by distilled water.
c) The slice was subjected to differentiation using 95% alcohol or 0.1% glacial acetic acid and was microscopically controlled to the extent that the nidus showed clearly.
d) The slice was rapidly dehydrated with anhydrous ethanol to obtain a product.
(e) The product was cleared with eco-friendly clearing agents, mounted, and examined by microscopic.

**Table 12 Number of neuronal Niehl' s bodies in the substantia nigra region of the midbrain of mice**

| Group | Group S | Group M | Group D | Group L |
|---|---|---|---|---|
| Number of Niehl's bodies | 64.8±6.0^{***} | 42.3±6.1 | 55.5±4.0^{***} | 57.8±6.5^{***} |

| | | | | |
|---|---|---|---|---|
| Note: compared to the model group (Group M), ^{***}P<0.001. Data are expressed as x+SD and multiple group comparison analysis is performed using ANOVA. | | | | |

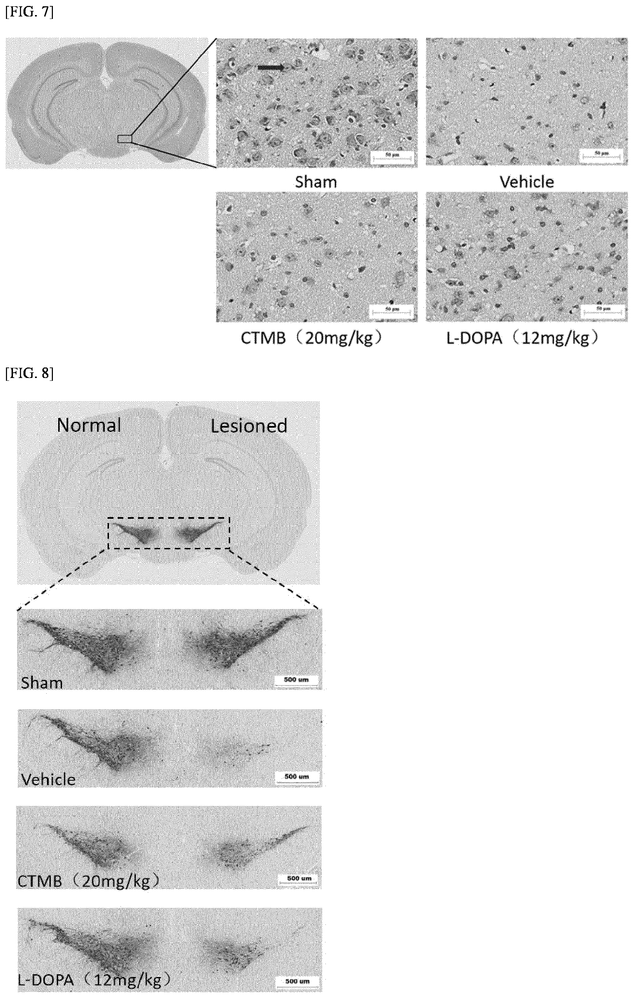

As shown in FIG. 5 and FIG. 6, at 6 weeks after injection of 6-OHDA, coagulative necrosis is found in the neuronal cells of the substantia nigra region and the striatum of the right side of the midbrain in the mice. The nuclei are inconspicuous, and there is a plurality of glial cells with diffuse or limited hyperplasia. Glial cells enter into the cytosol or protrusion of necrotic neuronal cells, forming the phenomenon of phagocytosis. In addition, the results of FIG. 7 and Table 12 show that a plurality of neurons in the substantia nigra region of the right side of the midbrain are crumpled and deeply stained, and the number of Niehl's bodies is reduced. The degree of pathological damage to the substantia nigra region and striatum is reduced in the CTMB group (group D) and levodopa group (group L) compared with the model group (group M), indicating that CTMB attenuates 6-OHDA-induced neuronal damage in the mouse substantia nigra and has neuroprotective effects.

7.2.3 Attenuating dopaminergic neuronal damage in the substantia nigra region of the midbrain in PD mice

Tyrosine hydroxylase (TH) protein expression was detected by immunohistochemistry. The operations were as follows.
a) De-paraffinization of Paraffin Sections to Water: paraffin slice was dewaxed sequentially in an environmental protection dewaxing agent (1), an environmental protection dewaxing agent (2), an environmental protection dewaxing agent (3) for 10 min, and then treated by anhydrous ethanol, 95% ethanol, and 75% ethanol for 5 min respectively, and washed by distilled water 3 times, followed by immersion washing.
b) Antigen repair.
c) Blocking of endogenous peroxidase: after the immersion washing, the repaired slice was immersed in 3% H₂O₂ and blocked for 30 min at room temperature in the dark and subjected to distilled water immersion washing.
d) Drawing circle: after the grouping pen drew the circle, the slice was placed in tris-buffered saline with tween 20 (TBST).
e) Closure: dropwise addition of 10% serum consistent with the source of the secondary antibody and incubation for 30 min at room temperature.
f) Incubation of primary antibody: the serum was removed, the primary antibody with 10% serum was diluted, the primary antibody working solution was prepared, 50-100 µL of the primary antibody working solution was add to each slice (depending on the size of the tissue), and the slice was incubated at 4°C overnight.
g) Incubation of secondary antibody: on the next day, the slice was taken out from the refrigerator, left at room temperature for 15 min (rewarming), rinsed 3 times with TBST, and then immersed 3 times for 3 min each time. The secondary antibody solution was diluted with TBST to formulate the secondary antibody working solution, 50-100µL (depending on the size of the tissue) of the secondary antibody working solution was added to each slice, and the slice was incubated at 37°C for 45 min. The slice was immersed 3 times by TBST, then immersed 3 times, with each time for 3 min.
h) DAB color development: the TBST was shaken off, 50-100 µL (depending on the size of the tissue) of freshly prepared DAB color development solution was added to each slice, a timer was used to record time, a microscope was used to observe the color development, the slice was washed with tap water to terminate the color development reaction and record the time of color development.

**Table 13 Effect of CTMB group on TH protein expression**

| Group | Group S | Group M | Group D | Group L |
|---|---|---|---|---|
| TH-positive neurons (a percentage of TH-positive neurons to uninjured side, %) | 95.6±5.3 | 10.3± 3.7^{###} | 39.9 ±12.1^{###, ***, &&} | 18.8± 7.6^{###} |

| | | | | |
|---|---|---|---|---|
| Note: Compared with the sham operation group (S group), ^{###}P<0.001; compared with the model group (M group), ***P<0.001; compared with the levodopa group (L group), ^{&&}P<0.01. Data are expressed as x±SD and multiple group comparison analysis is performed using ANOVA. | | | | |

As shown in FIG. 8 and Table 13, the expression of TH protein in the substantia nigra region on the injured side (right side) is significantly reduced after injection of 6-OHDA, i.e., the number of dopaminergic-positive neurons is significantly decreased (P<0.001). The CTMB group (group D) significantly inhibits 6-OHDA-induced apoptosis in dopaminergic neurons and is significantly better than the levodopa group (group L), suggesting that CTMB can slow down the progression of Parkinson's disease in animal models. The levodopa group (group L) also inhibits 6-OHDA-induced apoptosis in dopaminergic neurons, but there is no statistical difference.

### 7.2.4 Measurement of MDA and GSH contents and SOD activity in striatum

Malondialdehyde (MDA) is a commonly used indicator of membrane lipid peroxidation, reduced glutathione (GSH) is one of the most commonly used non-enzymatic indicators of antioxidant substances, and superoxide dismutase (SOD) is a common indicator of antioxidant enzymes. After completing the evaluation of motor function after 4 weeks of drug administration, the mice were deeply anesthetized and perfused with 20 mL of saline via the heart. The brain was rapidly severed to isolate the striatal region on the injured side (right side), weighed, and placed into a 1.5 mL EP tube, and homogenized at low temperature by adding phosphate-buffered saline (PBS) at the ratio of a weight of animal tissues (g) to a volume of homogenization medium (mL) of 1:9. Centrifugation was performed at 4°C, 10000×g for 10 min, and the supernatant was set to detect the contents of MDA and GSH, and SOD viability according to the instructions of the MDA, GSH and SOD colorimetric assay kit, respectively.

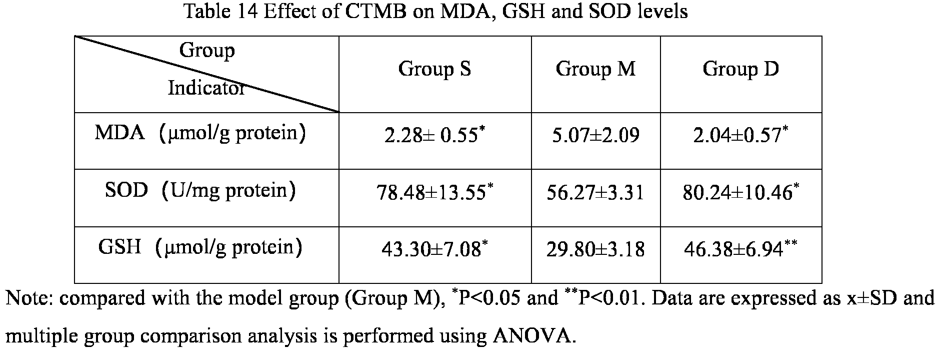

As shown in Table 14, MDA in the striatum of mice in the model group (group M) is significantly higher and GSH and SOD levels are significantly lower compared with the sham operation group (group S). The CTMB group (Group D) significantly reduces the concentration of MDA and significantly increases the concentration of GSH and SOD activity in the striatum of the model mice. The above results suggest that CTMB can exert anti-PD effect by improving oxidative stress.

The foregoing is only a preferred embodiment of the present disclosure, and it should be noted that, for a person of ordinary skill in the art, various improvements and embellishments may be made without departing from the principles of the present disclosure. These improvements and embellishments should also be regarded as the scope of protection of the present disclosure.

## Claims

1. A 1-(cyclobutylidenemethyl)-2, 4, 5-trimethoxybenzene compound, having a structural formula (I):

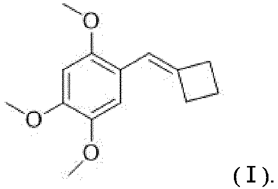

2. A method for preparing the 1-(cyclobutylidenemethyl)-2, 4, 5-trimethoxybenzene compound of claim 1, comprising:
(1) mixing a compound 1a with dichloromethane to obtain a mixture, and adding a compound 1b to the mixture for reaction to obtain a compound 1c;
(2) mixing the compound 1c, a compound 1d, and aluminum trichloride with methylene chloride in turn for reaction to obtain a compound 1e;
(3) mixing the compound 1e, sodium borohydride solution, and sodium hydroxide solution with tetrahydrofuran in turn for reaction to obtain a compound 1f; and
(4) mixing the compound 1f, sodium acetate, and acetic anhydride for reaction to obtain the 1-(cyclobutylidenemethyl)-2, 4, 5-trimethoxybenzene compound, wherein
the compound 1a is 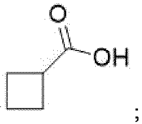 the compound 1b is 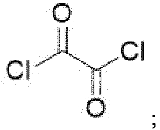 and the compound 1d is

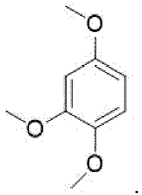

3. The method of claim 2, wherein in operation (1), a mass-volume-mass ratio of the compound 1a, dichloromethane, and the compound 1b is within a range of (23-27) g: (130-170) mL: (93-97) g.

4. The method of claim 2 or claim 3, wherein in operation (1), a reaction temperature of the reaction is within a range of 20-30°C, the reaction is carried out under a stirring condition, and the reaction is ended until no bubble is generated under the stirring condition.

5. The method of claim 4, wherein in operation (2), a mass-mass-mass-volume ratio of the compound 1c, the compound 1d, aluminum trichloride, and methylene chloride is within a range of (22-27) g: (30-33) g: (26-30) g: (80-120) mL; and
the compound 1c is

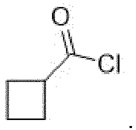

6. The method of any one of claims 2, 3, and 5, wherein in operation (2), a reaction temperature of the reaction is within a range of 20-30°C and a reaction time of the reaction is within a range of 2-3 h.

7. The method of claim 6, wherein in operation (3), a mass-volume-volume ratio of the compound 1e, the sodium borohydride solution, and tetrahydrofuran is within a range of (40-45) g: (18-22) mL: (140-160) mL, and a concentration of the sodium borohydride solution is within a range of 0.8-1.0 g/mL; and
the compound 1e is

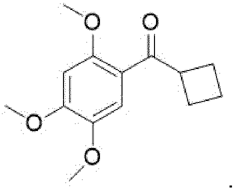

8. The method of claim 5 or claim 7, wherein in operation (3), a reaction temperature of the reaction is within a range of 50-65°C, and a reaction time of the reaction is within a range of 2-4 h.

9. The method of claim 8, wherein in operation (4), a mass-mass-volume ratio of the compound 1f, sodium acetate, and acetic anhydride is within a range of (38-45) g: (7-10) g: (200-230) mL;
the compound 1f is 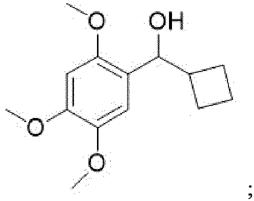 and
a reaction temperature of the reaction is within a range of 120-140°C, and a reaction time of the reaction is within a range of 2-4 h.

10. Use of the 1-(cyclobutylidenemethyl)-2, 4, 5-trimethoxybenzene compound of claim 1 in preparation of an antiepileptic drug.

11. Use of the 1-(cyclobutylidenemethyl)-2, 4, 5-trimethoxybenzene compound of claim 1 in preparation of a drug for treatment and/or prevention of traumatic craniocerebral injury disorders.

12. Use of the 1-(cyclobutylidenemethyl)-2, 4, 5-trimethoxybenzene compound of claim 1 in preparation of a drug for treatment and/or prevention of ischemic stroke.

13. Use of the 1-(cyclobutylmethyl)-2, 4, 5-trimethoxybenzene compound of claim 1 in preparation of a drug for treatment and/or prevention of hemorrhagic stroke.

14. Use of the 1-(cyclobutylidenemethyl)-2, 4, 5-trimethoxybenzene compound of claim 1 in preparation of a drug for prevention and/or treatment of Parkinson's disease.
